# EUROPÄISCHE PATENTSCHRIFT

(11) **EP 1 879 023 B1**
(45) Veröffentlichungstag und Bekanntmachung des Hinweises auf die Patenterteilung: **04.03.2015**
(21) Anmeldenummer: 07013537.1
(22) Anmeldetag: 11.07.2007
(51) Int. Cl.: G01N 27/414, G01N 33/00

(54) **Sensor zur Wasserstoff-Detektion**
Sensor for detecting hydrogen
Capteur destiné à la détection d'hydrogène

(30) Priorität: 14.07.2006 DE 102006033058
(43) Veröffentlichungstag der Anmeldung: 16.01.2008
(73) Patentinhaber: Micronas GmbH, 79108 Freiburg (DE)
(72) Erfinder: Widanarto, Wahyu, 85579 Neubiberg (DE); Knittel, Thorsten, 93080 Pentling-Großberg (DE); Senftleben, Oliver, 81539 München (DE); Senft, Christopher, 80798 München (DE); Eisele, Ignaz, 82057 Icking (DE)
(74) Vertreter: Koch Müller Patentanwaltsgesellschaft mbH

(56) Entgegenhaltungen:
- FLIETNER B ET AL: "Work function measurements for gas detection using tin oxide layers with a thickness between 1 and 200 nm" THIN SOLID FILMS SWITZERLAND, Bd. 250, Nr. 1-2, 1. Oktober 1994 (1994-10-01), Seiten 258-262, XP002455893 ISSN: 0040-6090
- GERGINTSCHEW Z ET AL: "The capacitively controlled field effect transistor (CCFET) as a new low power gas sensor" SENSORS AND ACTUATORS B, ELSEVIER SEQUOIA S.A., LAUSANNE, CH, Bd. 36, Nr. 1, Oktober 1996 (1996-10), Seiten 285-289, XP004061082 ISSN: 0925-4005
- KANG W P ET AL: "Performance and detection mechanism of a new class of catalyst (Pd, Pt, or Ag)-adsorptive oxide (SnOx or ZnO)-insulator-semiconductor gas sensors" SENSORS AND ACTUATORS B, ELSEVIER SEQUOIA S.A., LAUSANNE, CH, Bd. 22, Nr. 1, Oktober 1994 (1994-10), Seiten 47-55, XP004012398 ISSN: 0925-4005
- LORENZ H ET AL: "New suspended gate FET technology for physical deposition of chemically sensitive layers" SENSORS AND ACTUATORS A, ELSEVIER SEQUOIA S.A., LAUSANNE, CH, Bd. A23, Nr. 1 / 3, 1. April 1990 (1990-04-01), Seiten 1023-1026, XP000355786 ISSN: 0924-4247
- JEYAPRAKASH J D ET AL: "A simple route towards the reduction of surface conductivity in gas sensor devices" SENSORS AND ACTUATORS B, ELSEVIER SEQUOIA S.A., LAUSANNE, CH, Bd. 110, Nr. 2, Oktober 2005 (2005-10), Seiten 218-224, XP002455894 ISSN: 0925-4005
- DATABASE COMPENDEX [Online] ENGINEERING INFORMATION, INC., NEW YORK, NY, US; 2006, KANG K H ET AL: "MISFET type H2 sensor using Pd-black catalytic metal gate for high performance" XP002455895 Database accession no. E2006169827070 & 208TH MEETING OF THE ELECTROCHEMICAL SOCIETY - MEETING ABSTRACTS 2005, Bd. MA 2005-02, 2005, Seite 43, ISSN: 1091-8213
- LUNDSTROM I ET AL: "A hydrogen-sensitive MOS field-effect transistor" APPLIED PHYSICS LETTERS, AIP, AMERICAN INSTITUTE OF PHYSICS, MELVILLE, NY, US, Bd. 26, Nr. 2, 15. Januar 1975 (1975-01-15), Seiten 55-57, XP002086960 ISSN: 0003-6951
- BURGMAIR M ET AL: "Field effect transducers for work function gas measurements: device improvements and comparison of performance", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, vol. 95, no. 1-3, 15 October 2003 (2003-10-15), pages 183-188, XP004454668, ISSN: 0925-4005, DOI: 10.1016/S0925-4005(03)00412-X

## Beschreibung

Gassensor nach dem Prinzip der Austrittsarbeitsmessung zur Bestimmung von Wasserstoff.

Die Erfindung bezieht sich auf einen Sensor zur Wasserstoff-Detektion auf Basis eines Feldeffekttransistors gemäß den oberbegrifflichen Merkmafen des Patentanspruchs 1.

Aus "THIN SOLID FILMS, Bd. 250, Nr. 1-2, 1. Oktober 1994, Seiten 258-262; ISSN 0040-6090, Flietner B. et al: "Work function measurements for gas detection using tin oxide layers with a thickness between 1 an 200 nm. " und aus "SENSORS AND ACTUATORS B, Bd. 22, Nr. 1 October 1994, Seiten 47-55, ISSN 0925-4005; KANG W. P. et al: "Performance and detection mechanism of a new class of catalyst (Pd, Pt, or Ag)-adsorptive oxide (SnOx or ZnO)-insulator-semiconductor gas sensors" und aus SENSORS AND ACTUATORS A, Bd. A23, Nr. 1 / 3, 1. April 1990, Seiten 1023-1026; ISSN 0924-4247; Lorentz H. et al: "New suspended gate FET technology for physical deposition of chemically sensitive layers" sind gassensitive Schichten bekannt.

Zur Wasserstoff Detektion sind Sensoren bekannt, welche aus einem Feldeffekttransistor mit elektrisch schwebendem Gate und einem Gate mit einer Sensorschicht als sensitiver Schich: zum Detektieren von in einem Messraum befindlichem Wasserstoff ausgebildet sind. Der Einsatz von Platin für die Sensorschicht ist zum Ausbilden einer chemisch sensitiven Schicht als der Sensorschicht zur Wasserstoff-Detektion bekannt. Nachteilhafterweise weisen solche Sensoren bei Raumtemperatur eine schlechte Selektivität auf und sind bei zunehmender Betriebstemperatur nicht stabil. Bekannt ist ein Feldeffekttransistor mit schwebendem Gate mit einer hybrid-montierten gas-sensitiven oberen Elektrode aus I. Eisele, T. Doll, M. Burgmair, Low power gas detection with FET-Sensors, Sensors and Actuators B 78 (2001) 19-25 und M. Burgmair, H.-P. Frerichs, M. Zimmer, M. Lehmann, I. Eisele, Field. Effect Tranducers for Work Function Gas Measurements: Device Improvements and Comparison of Performance, Sensors and Actuators, B 95 (2003) 183-188. Physisorption oder Chemisorption an der Schicht an der oberen Elektrode oder dem Gate kann eine reversible Potentialdifferenz erzeugen. Die Potentialdifferenz bewirkt Änderungen eines Source-Drain-Stromes eines konventionellen p-Kanal-MOSFET über einen kapazitiven Spannungsteiler und ein schwebendes Gate.

Diese Art von Sensoren sind auch unter dem Begriff Suspended-Gate Sensoren bekannt. Generell handelt es sich um die Messung von Austrittsarbeitsänderungen an der sensitiven Schicht durch Messung des Einflusses auf den Kanal eines Feldeffekttransistors. Der Feldeffekttransistor kann als konventioneller SGFET oder als CCFET ausgelegt sein, wie dies bekannt ist aus I. Eisele, T. Doll, M. Burgmair, Low power gas detection with FET-Sensors, Sensors and Actuators B 78 (2001) 19-25 und M. Burgmair, H.-P. Frerichs, M. Zimmer, M. Lehmann, I. Eisele, Field Effect Tranducers for Work Function Gas Measurements: Device Improvements and Comparison of Performance, Sensors and Actuators, B 95 (2003) 183-188. Im folgenden wird dies als Suspended-Gate FETs (SGFETs) bezeichnet.

Des weiteren gibt es die sogenannten Lundström-Sensoren bei denen die sensitive Schicht (Palladium) direkt auf einen Isolator eines Feldeffekttransistors aufgebracht ist. Der Wasserstoff dissoziert an der Oberfläche und die H2-Atome lagern sich an der Grenzfläche zum Isolator an. Auch hierbei handelt es sich um eine Messung der Austrittsarbeit.

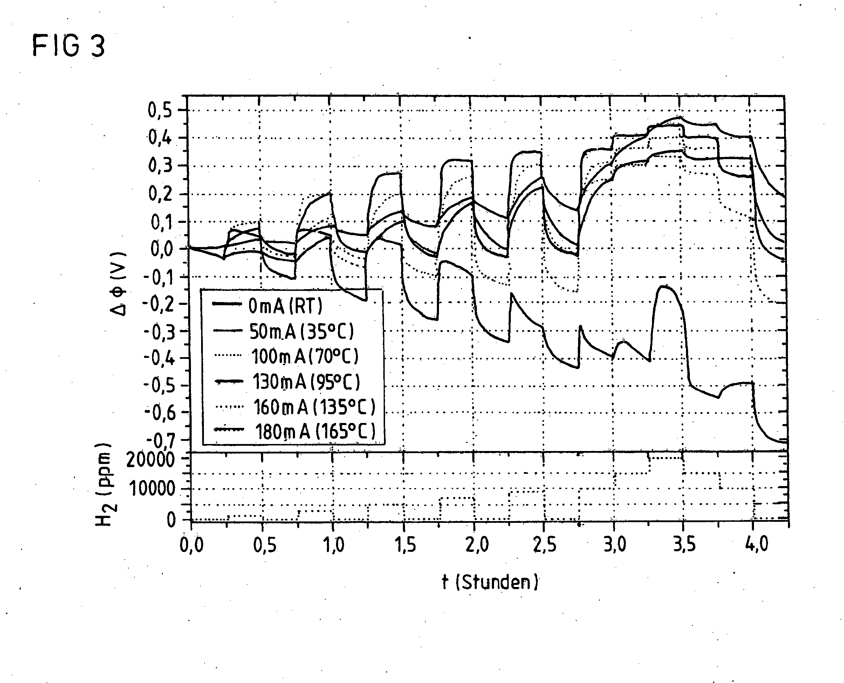

Anhand Fig. 3 ist u.a. eine Interaktion bzw. Wechselwirkung zwischen Wasserstoff und einer Platin-Oberfläche dargestellt, welche in hohem Maße von der Temperatur und Luftfeuchtigkeit abhängt, wie dies aus K. Scharnagl, Feldeffekttransistoren mit Luftspalt für den Nachweis von Wasserstoff, Dissertation der Fakultät für Elektrotechnik und Informationstechnik der UniBW München, 2002, bekannt ist. Eine mögliche Erklärung dafür besteht darin, dass die Wechselwirkung zwischen Platinoxid an der Oberfläche mit Wasserstoffmolekülen (H₂) bei Raumtemperatur OH-Gruppen während eines Übergangsprozesses erzeugt, welcher zu synthetischem Wasser (H₂O) führt. Das Dipolmoment von OH-Gruppen und das synthetische Wasser erzeugen eine Arbeitsfunktionsänderung der Oberfläche, d.h. eine Änderung der Austrittsarbeit ΔΦ der sensitiven Schicht und/oder Oberfläche, welche zu einem Sensorsignal führt. Das Erhöhen der Betriebstemperatur bewirkt, dass das Wasser verdampft, was Sauerstoff an der Oberfläche vermehrt auftreten lässt. Derartige Sauerstoff-Vakanzen werden durch Wasserstoff-Moleküle gefüllt, was zu einer dortigen Reaktion mit Pt-O in der Form Pt-OH führt. Dies bedeutet, dass Wasserstoffmoleküle (H₂) an der Oberfläche chemisch adsorbiert werden, was als Chemisorption bezeichnet wird.

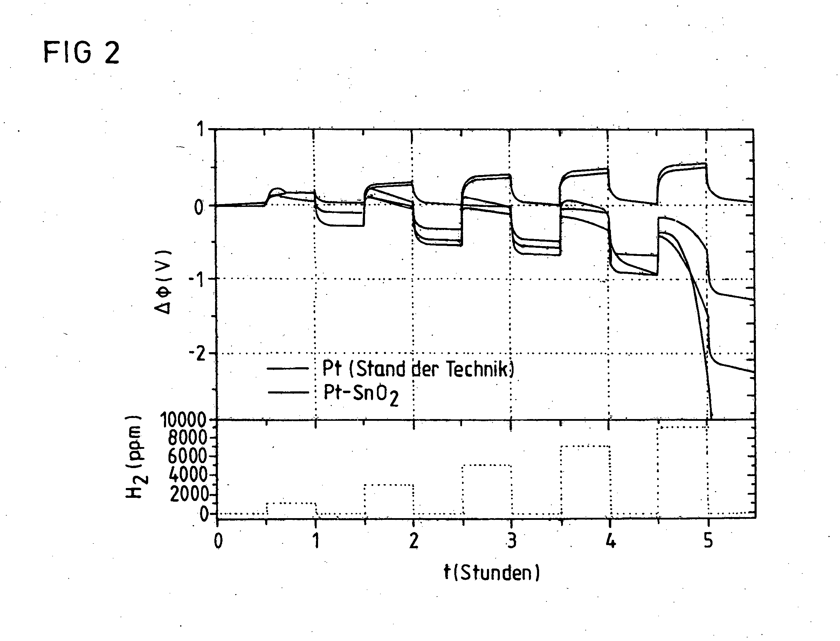

Mit Blick auf Aspekte des Standes der Technik ist Fig. 2 dabei natürlich nur hinsichtlich der Kurve für eine reine Elektrode aus Platin Pt zu betrachten, welche als Relative zur ursprünglichen Austrittsarbeit ΔΦ in Volt, d.h. der Austrittsarbeit ohne Gaseinwirkung, über der Zeit t aufgetragen ist.

Die Aufgabe der Erfindung besteht darin, einen Sensor zur Wasserstoff-Detektion auf Basis eines Feldeffekttransistors mit elektrisch schwebendem Gate derart zu verbessern, dass Messungen durch andere Stoffe als Wasserstoff, insbesondere durch Wasser, und vorzugsweise auch durch Temperaturschwankungen weniger beeinträchtigt werden.

Diese Aufgabe wird durch den Sensor mit den Merkmalen des Patentanspruchs 1 bzw. die Verwendung eines solchen Sensors gemäß den Merkmalen des Patentanspruchs 9 gelöst. Vorteilhafte Ausgestaltungen sind Gegenstand von abhängigen Ansprüchen.

Verwendet wird demgemäß ein Gassensor nach dem Prinzip der Austrittsarbeitsmessung mit einer Sensorschicht zur Bestimmung von in einem Messraum befindlichem Wasserstoff, wobei zwischen der Sensorschicht und dem Messraum eine SnO₂ aufweisende Schicht angebracht ist.

Die Sensorschicht ist als ei Teil eines Feldeffekt-Transistors ausgebildet. Der Feldeffekt-Transistor ist ein Suspended Gate Feldeffekttransistor

Verwendet wird somit als Gassensor insbesondere ein Sensor zur Wasserstoff-Detektion, der einen Feldeffekttransistor elektrisch mit schwebendem Gate und ein Gate mit einer Sensorschicht als sensitiver Schicht zum Detektieren von in einem Messraum befindlichem Wasserstoff aufweist. Vorteilhaft wird der Sensor dadurch, dass die Sensorschicht gegenüber dem Messraum durch eine SnO₂ aufweisende Schicht getrennt ist.

Unter Wasserstoff sind dabei insbesondere Wasserstoffmoleküle zu verstehen. Die Trennschicht kann insbesondere auch ganz aus SnO₂ ausgebildet sein. Gegebenenfalls können zwischen der Trennschicht und der Sensorschicht oder zwischen der Sensorschicht und einer diese tragenden Deckplatte des Gates auch weitere Schichten als Hilfsschichten ausgebildet sein, welche zur Verbindung dieser Komponenten aneinander dienen.

Die SnO2 Schicht hat bevorzugt eine Dimension von < 100 nm, vorzugsweise < 50 nm.

Die Trennschicht ist aus SnO₂ mit einer derart dimensionierten Permeabilität ausgebildet, dass Wasserstoffmoleküle zu der Sensorschicht hindurchgelassen und dadurch dort reagieren können bzw. detektiert werden können. Unter Permeabilität wird die Eignung eines Materials verstanden, einen Stoff hindurchzulassen, wobei diese Eignung oftmals auch als Porosität bezeichnet wird oder bei bestimmten Porositäten angenommen wird.

Die Sensorschicht weist bevorzugt Platin und/oder eine Platin-Sauerstoff-Verbindung, insbesondere eine Platin-Hydroxid-Gruppe, auf oder ist daraus ausgebildet, wie dies für sich genommen von derartigen Sensoren zur Wasserstoff-Detektion bekannt ist.

Der Messraum ist als ein Spalt zwischen dem Gate mit der Sensorschicht und der Trennschicht einerseits und andererseits dem schwebenden Gate ausgebildet, so dass ein definierter Zuführungsraum gebildet wird, welchem das zu untersuchende Medium zugeführt wird.

Die Trennschicht ist als eine SnO₂-Schicht ausgebildet zum Aufnehmen von Wasser an ihrer SnO₂-Oberfläche oder in derem porösen Volumen. Unter Wasser ist allgemein auch Wasserdampf zu verstehen.

Vorteilhaft ist entsprechend die Verwendung eines solchen Sensors zum Detektieren von Wasserstoff in einem trockenem oder feuchten Gasgemisch, in einem Fluid und/oder in einer Flüssigkeit. Insbesondere sind mit einem derartigen Sensor auch zu untersuchende Medien mit weiteren Bestandteilen neben Wasserstoff und Wasser untersuchbar hinsichtlich des Wasserstoffgehalts. Gemäß erster Versuche ist die Verwendung eines solchen Sensors in einem Temperaturbereich von 0°C - 200°C mit verringerter oder sogar ganz ohne eine Beeinträchtigung durch Wasseranteile in dem zu untersuchenden Medium vorteilhaft möglich. Jedoch ist die Anwendung nicht auf diesen Temperaturbereich beschränkt.

Der Grundgedanke des bevorzugten Sensors, welcher die Empfindlichkeit gegenüber Wasser und Temperaturschwankungen reduziert und die gewünschte Wechselwirkung beibehalten lässt, besteht somit darin, dass eine vorzugsweise poröse SnO₂-Schicht auf die Oberfläche des Gates bzw. die Oberfläche der chemisch-sensitiven Schicht des Gates aufgetragen wird, um Sauerstoff zu erzeugen, welcher auf der Oberfläche bleibt. Aufgrund der geringen Dimension von Wasserstoffmolekülen können diese noch durch diese durch SnO₂ ausgebildete Trennschicht hindurch zur Oberfläche der sensitiven Schicht diffundieren und mit dem Pt-O wechselwirken.

Somit werden eine oder mehrere poröse Zinnoxid-Schichten auf dem Platin oder der Platin aufweisenden Sensorschicht abgeschieden, um eine oder mehrere chemisch sensitive Elektroden in einem zur Gas-Detektion, insbesondere Wasserstoff-Detektion, geeigneten Feldeffekttransistor mit schwebendem Gate auszubilden. Im Rahmen erster Versuche wurden Änderungen von Betriebsfunktionen, d.h. der Basislinie des Sensors hinsichtlich verschiedener Wasserstoff-Konzentrationen als eine Funktion von Temperatur und Feuchtigkeit gemessen. Die Ergebnisse zeigten, dass SnO₂ sehr gut geeignet ist, um die Sensorsignalantwort zwischen Raumtemperatur und 135°C zu stabilisieren. Die Antwortzeit ist dabei sehr schnell (t₅₀ < 10 s) und die Wechselwirkungssensitivität (engl. cross sensitivity) wird im Vergleich zur Verwendung von reinem Platin reduziert. Ein Experiment hinsichtlich Langzeit-Stabilität zeigte, dass Signal strukturen der Sensoren auch zwei Monate nach einer ersten Konditionierung stabil verblieben.

Ein Ausführungsbeispiel wird nachfolgend anhand der Zeichnung näher erläutert. Es zeigen:
- Fig. 1: eine Schnittdarstellung durch einen Sensor zur Wasserstoff-Detektion mit einem Feldeffekttransistor mit schwebendem Gate;
- Fig. 2: einen Vergleich der Sensivitäts-Charakteristik zwischen Sensoren mit und ohne porösem SnO₂ bei 95°C unter trockenen Bedingungen;
- Fig. 3: entsprechende Diagramme hinsichtlich eines Temperatur-Effekts auf die Sensoren mit Pt-SnO₂ bei trockenen Bedingungen;
- Fig. 4: eine Selektivität der Sensoren mit Pt-SnO₂ bei 95°C unter trockenen Bedingungen;
- Fig. 5: eine Antwort- bzw. Reaktionszeit der Pt-SnO₂-Sensoren bei Betriebstemperatur von 95°C unter trockenen bzw. humiden (30% r.h.) Bedingungen und
- Fig. 6: eine Langzeit-Stabilität der Pt-SnO₂-Sensoren bei 10.000 ppm H₂-Gasexposition bei 95°C unter trockenen Bedingungen nach zwei Monaten.

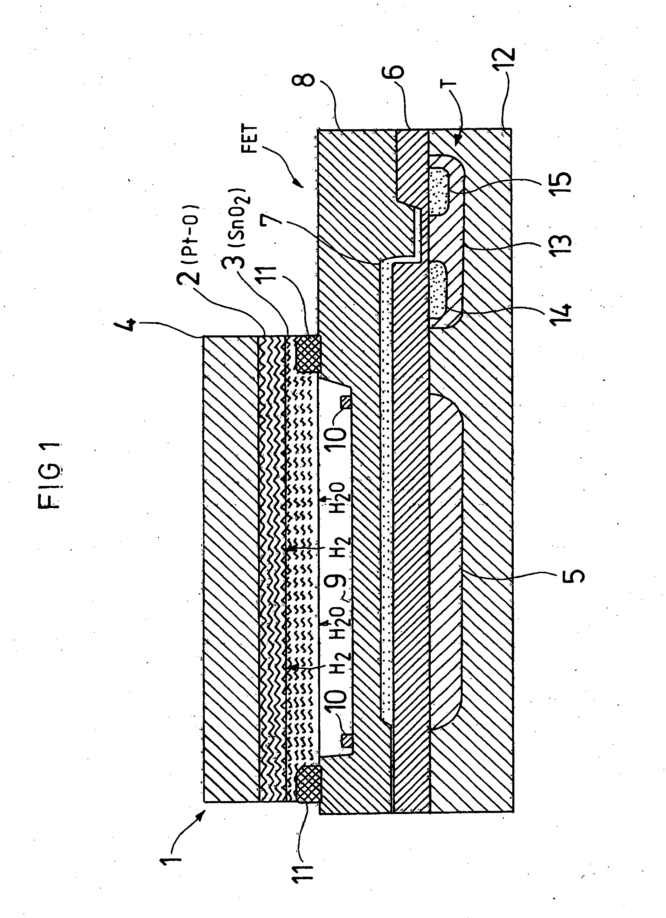

Wie aus Fig. 1 ersichtlich, besteht eine bevorzugte Sensoranordnung eines Sensors zur Wasserstoff-Detektion aus einem Gate 1 mit einer Sensorschicht 2, welche vorzugsweise eine Platin-Sauerstoff-Verbindung aufweist oder aus einer solchen besteht, sowie einer Trennschicht 3 aus porösem Zinnoxid in der Form SnO₂, wobei ein solches Gate 1 oberhalb weiterer Komponenten eines Feldeffekttransistors FET angeordnet ist.

Das Gate 1 weist dabei vorzugsweise eine elektrisch leitfähige Gate-Platte 4 auf, welche in der Zeichnung an ihrer Unterseite die Sensorschicht 2 und die Trennschicht 3 trägt. Die Gate-Platte 4 ist zur Umgebung isoliert und ermöglicht die Verschiebung der Einsatzspannung eines Auslesetransistors, wenn sich die Ladungsverteilung auf ihr ändert.

Als eine weitere Anschluss-Elektrode ist eine Kontroll-Elektrode 5 parallel zu dem Gate 1, welches die Gate-Elektrode ausbildet, in dem Feldeffekttransistor FET angeordnet. Zwischen der Kontroll-Elektrode 5 und dem Gate 1 sind eine SiO₂-Schicht 6, eine Elektrode zum Ausbilden des schwebenden Gates 7 und eine Passivierungsschicht 8 sowie ein Messraum 9 in Form beispielsweise eines Luftspalts ausgebildet.

Der Messraum 9 dient zum Hindurchleiten des Mediums, welches hinsichtlich seines Wasserstoffgehalts zu untersuchen ist. Der Messraum 9 wird durch eine Vertiefung in der Oberfläche der Passivierungsschicht 8 ausgebildet und weist von den seitlichen Flanken des Messraums 9 etwas beabstandet einen Schutzring (engl. guard ring) in für sich bekannter Art und weise auf.

Auf der Oberfläche der Passivierungsschicht 8 um den Messraum 9 außenseitig herum ist das Gate 1 mit der dem Messraum 9 zugewandten Trennschicht 3 befestigt. Insbesondere ist das Gate 1 mittels eines Klebers 11 im Umfangsbereich des Messraums 9 angeklebt.

Die Kontroll-Elektrode 5 ist unterhalb der SiO₂-Schicht 6 in einer Basis 12 ausgebildet. Seitlich beabstandet von der Kontroll-Elektrode 5 ist in der Basis unterhalb der SiO₂-Schicht 6 eine Transistor-Anordnung ausgebildet, welche eine Basisschicht bzw. ein Substrat 13 aufweist, welches Drain 14 und Source 15 des Feldeffekttransistors FET umgreift. Drain 14 und Source 15 sowie dazwischen das Substrat 13 grenzen an der SiO₂-Schicht 6 an. Diesem mittleren Abschnitt des Substrats 13 zwischen Drain 14 und Source 15 liegt ein seitlicher Abschnitt der als schwebendem Gate 7 ausgebildeten Elektrode als Gate-Anschluss gegenüber.

Fig. 2 zeigt vorteilhaft einen Vergleich der Stabilitäts-Charakteristika zwischen einem Sensor gemäß dem Stand der Technik mit einer reinen Platin-Schicht oder Platin-OxidSchicht am Gate 1 gegenüber der gemäß Fig. 1 bevorzugten Ausgestaltung mit der darüber angeordneten Trennschicht 3 aus porösem SnO₂ bei 95°C unter trockenen Betriebsbedingungen.

Fig. 3 zeigt, dass das Sensorsignal unter trockenen Bedingungen bis zu Temperaturen von zumindest etwa 135°C stabil ist. Ein Erhöhen der Temperatur bewirkt das Verdampfen synthetischen Wassers in dem Messraum 9, was viele Sauerstoff-Vakanzen an der Oberfläche erzeugt. Da SauerstoffAtome und deren zugeordnete Vakanzen an dem porösen SnO₂ relativ mobil sind, wie dies für sich genommen aus W. Hellmich, G. Müller, Ch. Bosch-v. Braunmühl, T. Doll, I. Eisele, Field-Effect-Induced Gas Sensitivity Changes in Metal Oxides, Sensors and Actuators B 43 (1997) 132-139 bekannt ist, zieht die Oberfläche Sauerstoff aus dem SnO₂, um Pt-Oxid an der Oberfläche zu halten. Die beste Sensorantwort auf Wasserstoffmoleküle H₂ wird bei den ersten Versuchen bei einer Betriebstemperatur von 95°C erzielt. Oberhalb von 135°C wird eine Signaldrift beobachtet, da die Sauerstoffzufuhr aus dem SnO₂ bei den Dimensionierungen gemäß dem ersten versuchsweisen Aufbau nicht mehr ausreichend ist.

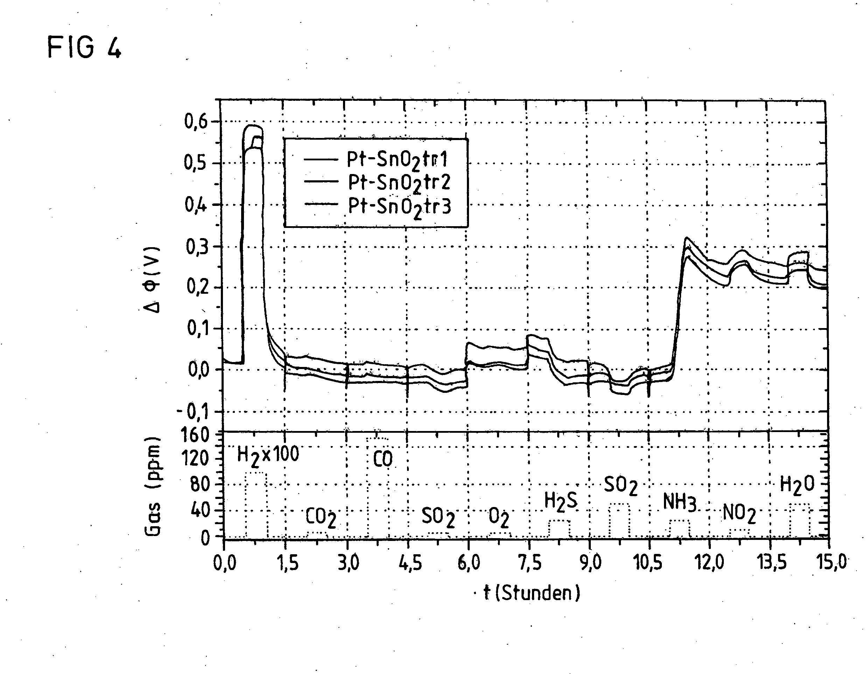

Derartige Sensoren mit Pt-SnO₂ zeigen insbesondere bei 95°C unter trockenen Bedingungen eine sehr gute Selektivität und Stabilität. Auch wenn verschiedene Gase in den Sensor eingebracht werden, zeigen sich keine signifikanten Signaländerungen, Fig. 4 zeigt eine Selektivität der Sensoren entsprechend mit Pt/SnO₂ bei 95°C unter trockenen Bedingungen.

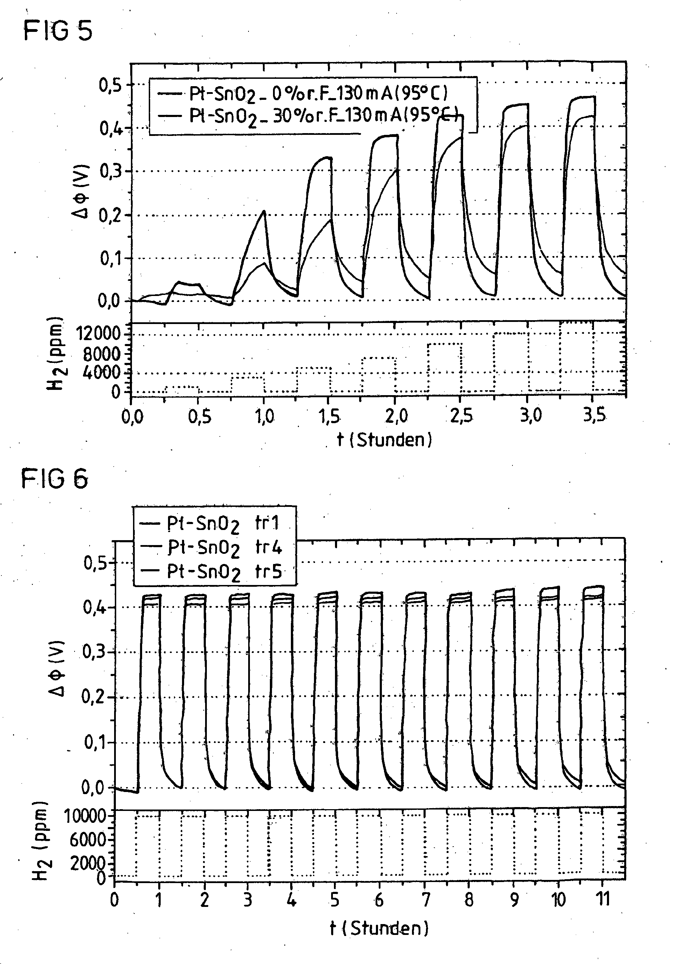

Aus den Kurven gemäß Fig. 5 ist ersichtlich, dass die Austrittsarbeit ΔΦ mit zunehmender relativer Humidität bzw. Feuchtigkeit bei jeder Konzentration von Wasserstoffmolekülen H₂ abnimmt. Trotzdem ist die Durchführung von zuverlässigen Messungen noch möglich. Unter humiden Bedingungen wird Wasserdampf von der SnO₂-Oberfläche adsorbiert. OH-SN₍ₛ₎-Bonds und O₍ₛ₎H-Gruppen, welche bei dem porösen SnO₂ ausgebildet werden, bewirken eine Sensitivitäts-Verschlechterung des Sensors, da sie H₂-Moleküle ziehen, um synthetisches Wasser an dem porösen SnO₂ auszubilden, so dass nur wenige H₂-Moleküle die Platin-Oberfläche der Sensorschicht 2 erreichen können. Für eine Wechselwirkung zwischen Wasserdampf H₂O an der Oberfläche der Sensorschicht 2 kann die Reaktion postuliert werden gemäß

2H₂O₍ₗ₎ + SnO₂₍ₛ₎ ↔ (OH - Sn₍ₛ₎) + 30₍ₛ₎H.

Anhand Fig. 6 ist eine Langzeit-Stabilität derartiger Sensoren anhand der dargestellten Kurven gut erkennbar. Derartige stabile Signale über eine Dauer von zwei Monaten zeigen, dass poröses SnO₂ die Platin-Oberfläche vor Angriffen aufgrund einer Wasserstoff-Chemisorption schützen.

Zusammengefasst kann festgestellt werden, dass eine Sauerstoffzufuhr aus porösem SnO₂ der Trennschicht 3 geeignet ist, die Wasserstoff-Antwort von Platin-Oberflächen in Feldeffekttransistoren FET mit schwebendem Gate 7 signifikant zu stabilisieren.

## Patentansprüche

1. Gassensor nach dem Prinzip der Austrittsarbeitsmessung mit einer Sensorschicht (2) zur Bestimmung von in einem Messraum (9) befindlichem Wasserstoff,
zwischen der Sensorschicht (2) und dem Messraum (9) eine SnO₂ aufweisende Schicht angebracht ist und
**dadurch gekennzeichnet, dass**
bei dem der Messraum (9) als ein Spalt zwischen einem Gate (1) mit der Sensorschicht (2) und der SnO₂ aufweisenden Schicht des Feldeffekt-Transistors (FET) einerseits und andererseits dem schwebenden Gate (7) des Feldeffekt-Transistors (FET) ausgebildet ist, und
bei dem die SnO₂-Schicht (3) ausgebildet ist zum Aufnehmen von Wasser (H₂O) an ihrer SnO₂-Oberfläche oder in derem porösen Volumen.

2. Gassensor nach Anspruch 1, bei dem die Sensorschicht (2) ein Teil eines Feldeffekt-Transistors (FET) ist.

3. Gassensor nach Anspruch 2, bei dem der Feldeffekt-Transistor (FET) ein Suspended Gate Feldeffekttransistor ist.

4. Gassensor nach einem vorstehenden Anspruch, bei dem die das SnO₂ aufweisende Schicht eine Dicke von weniger als 100 nm, insbesondere von weniger als 50 nm aufweist.

5. Gassensor nach einem vorstehenden Anspruch, bei dem die das SnO₂ aufweisende Schicht (2) als eine SnO₂-Trennschicht (3) ausgebildet ist.

6. Gassensor nach einem vorstehenden Anspruch, bei dem die SnO₂ aufweisende Schicht mit einer derart dimensionierten Permeabilität ausgeblldet ist, dass Wasserstoffmoleküle zu der Sensorschicht (2) hindurchgelassen werden.

7. Sensor nach einem vorstehenden Anspruch, bei dem die Sensorschicht (2) ein Edelmetall aufweist, insbesondere Palladium, Platin und/oder eine Platin-Sauerstoff-Verbindung (Pt-O), insbesondere eine Platin-Hydroxid-Gruppe, aufweist oder daraus ausgebildet ist.

8. Verwendung eines Sensors nach einem vorstehenden Anspruch zum Detektieren von Wasserstoff in einem trockenen oder feuchten Gasgemisch.

9. Verwendung des Sensors nach Anspruch 11 in einem Temperaturbereich von 0°C - 200°C, insbesondere in einem Temperaturbereich von 0°C - 140°C.

## Claims

1. Gas sensor according to the principle of work-function measurement with a sensor layer (2) for determining hydrogen present in a measuring space (9), a layer comprising SnO₂ being mounted between the sensor layer (2) and the measuring space (9),
**characterised in that**
the measuring space (9) is formed as a gap between a gate (1) with the sensor layer (2) and the layer, which comprises SnO₂, of the field effect transistor (FET) on the one hand and the floating gate (7) of the field effect transistor (FET) on the other hand and
the SnO₂ layer (3) is constructed for the reception of water (H₂O) at its SnO₂ surface or in the porous volume thereof.

2. Gas sensor according to claim 1, in which the sensor layer (2) is a part of a field effect transistor (FET).

3. Gas sensor according to claim 2, in which the field effect transistor (FET) is a suspended-gate field effect transistor.

4. Gas sensor according to any one of the preceding claims, in which the layer comprising the SnO₂ has a thickness of less than 100 nm, particularly less than 50 nm.

5. Gas sensor according to any one of the preceding claims, in which the layer (2) comprising the SnO₂ is constructed as an SnO₂ separating layer (3).

6. Gas sensor according to any one of the preceding claims, in which the layer comprising the SnO₂ is constructed with a permeability so dimensioned that the hydrogen molecules are let through to the sensor layer (2).

7. Sensor according to any one of the preceding claims, in which the sensor layer (2) comprises a noble metal, particularly palladium, platinum and/or a platinum-oxygen compound (Pt-O), particularly a platinum-hydroxide group, or is constructed therefrom.

8. Use of a sensor according to any one of the preceding claims for detection of hydrogen in a dry or moist gas mixture.

9. Use of the sensor according to claim 11 in a temperature range 0° C to 200° C, particularly in a temperature range of 0° C to 140° C.

## Revendications

1. Détecteur de gaz opérant selon le principe de la mesure du travail de sortie avec une couche de détecteur (2), destiné à la détermination d'hydrogène se trouvant dans une chambre de mesure (9), une couche contenant du SnO₂ étant placée entre la couche de détecteur (2) et la chambre de mesure (9), **caractérisé en ce que** la chambre de mesure (9) est réalisée sous la forme d'une fente entre une grille (1) avec la couche de détecteur (2) et la couche contenant du SnO₂ du transistor à effet de champ (FET) d'une part et d'autre part la grille flottante (7) du transistor à effet de champ (FET), et dans lequel la couche de SnO₂ (3) est conçue de façon à capter l'eau (H₂O) à sa surface de SnO₂ ou dans le volume poreux de celle-ci.

2. Détecteur de gaz selon la revendication 1, dans lequel la couche de détecteur (2) fait partie d'un transistor à effet de champ (FET).

3. Détecteur de gaz selon la revendication 2, dans lequel le transistor à effet de champ (FET) est un transistor à effet de champ à grille suspendue.

4. Détecteur de gaz selon une revendication précédente, dans lequel la couche contenant le SnO₂ présente une épaisseur de moins de 100 nm, en particulier de moins de 50 nm.

5. Détecteur de gaz selon une revendication précédente, dans lequel la couche contenant le SnO₂ (2) est réalisée sous la forme d'une couche de séparation en SnO₂ (3).

6. Détecteur de gaz selon une revendication précédente, dans lequel la couche contenant du SnO₂ est réalisée avec une perméabilité dimensionnée de telle manière que des molécules d'hydrogène puissent traverser jusqu'à la couche de détecteur (2).

7. Détecteur selon une revendication précédente, dans lequel la couche de détecteur (2) présente un métal noble, en particulier du palladium, du platine et/ou un composé de platine-oxygène (Pt-O), en particulier un groupe hydroxyde de platine, ou en est constituée.

8. Utilisation d'un détecteur selon une revendication précédente pour la détection d'hydrogène dans un mélange de gaz sec ou humide.

9. Utilisation du détecteur selon la revendication 11 dans un domaine de température de 0°C à 200°C, en particulier dans un domaine de température de 0°C à 140°C.
